# EUROPEAN PATENT SPECIFICATION

(11) **EP 4 138 743 B1**
(45) Date of publication and mention of the grant of the patent: **25.11.2026**
(21) Application number: 21717915.9
(22) Date of filing: 15.04.2021
(51) Int. Cl.: A61F 5/445

(54) **CONTINUITY DEVICE FOR DERIVATIVE ILEOSTOMY**
KONTINUITÄTSVORRICHTUNG FÜR ABLEITENDE ILEOSTOMIE
DISPOSITIF DE CONTINUITÉ POUR ILÉOSTOMIE DÉRIVÉE

(30) Priority: 22.04.2020 EP 20382330
(43) Date of publication of application: 01.03.2023
(73) Proprietor: Servizo Galego de Saude, 15703 Santiago de Compostela (ES)
(72) Inventor: NOGUERA AGUILAR, José Francisco, 15006 A Coruña (ES); AGUIRREZABALAGA GONZÁLEZ, Javier Antonio, 15006 A Coruña (ES); CENTENO CORTES, Alberto, 15006 A Coruña (ES)
(74) Representative: Hoffmann Eitle
(86) International application number: PCT/EP2021/059778
(87) International publication number: WO 2021/213887

(56) References cited:
- WO-A1-2019/171335
- CN-A- 107 625 573
- CN-U- 209 154 851
- JP-A- H05 212 071
- US-A1- 2019 015 241

## Description

### Field and object of the invention

The present invention generally relates to stomal medical devices for treating patients affected by colorectal cancer.

More specifically, an object of the invention is to provide a continuity device that enable to obtain preoperative predictive test to know if the results of the derivative ileostomy closure surgery will be as expected, and to predict the correct anorectal functioning after the digestive reconstruction surgery.

An additional object of the invention is to provide continuity device to prepare the efferent loop of the ileostomy for the reconstruction surgery, avoiding intestinal atrophy and improving the caliber of the loop for the subsequent ileoileal anastomosis, as well as the conditioning prior to bowel reconstruction to improve distal bowel capacity and function.

### Background of the invention

Colorectal cancer represents 9% of all cancers around the globe. It is the third most common cause of death in the USA. In 2016, 158,000 new cases of colorectal cancer were recorded. There are similar incidence rates for cancer of the colon in both sexes, and a slight male predominance for rectal cancer. In the UK, colorectal cancer is the second most common cause of death, with 20,000 new cases every year. In addition, in many countries around the world, including European countries, rates of colorectal cancer are high.

Rectal cancer shows a growing trend. Classically, it accounted for one third of patients born with colorectal cancer, but nowadays it is estimated to account for 40% of CRC and with a growing trend. Moreover, this cancer is affecting younger patients in a selective way, which means a greater interest in sphincter preservation and a good posterior functionality. This encourages the research in order to improve the quality of this process of improvement of the surgery of reestablishment of the colorectal continuity after a derivative ileosotomy.

In middle and low anterior resection for rectal cancer (66% of rectal cancer), protective ileostomy is strongly suggested when anastomosis is performed less than 8 - 10 cm from the anal verge or when a previous treatment with radiotherapy was administrated. The construction of a protective stoma significantly reduces the incidence of anastomotic leakage and re-operation but it may cause another related problems due to the bowel absence of transit and the surgical closure. In many patients the closure of this ileostomy is delayed for months due to postoperative treatments and the long waiting lists presented by the public health system.

Most of these patients are carriers of loop ileostomy because they have previously had a rectal resection with low colorectal anastomosis that usually leads to the so-called "anterior resection syndrome", with the possibility of postoperative anal incontinence and anorectal dysfunction, sometimes very difficult to solve.

The loop-ileostomy is a derivative stoma to prevent postoperative anastomotic problems in colorectal surgery. This preventive stoma is being performed more and more frequently due to the advancement in surgical rectal techniques. This surgical advancement makes possible to save the distal part of the rectum and the anus.

Decades ago the majority of stomas were colic (colostomies) while nowadays we are changing colic stomas for ileal stomas (ileostomy), whose function is to protect a colorectal anastomosis from high risk of dehiscence due to the proximity of the anorectal sphincter system.

Patients who are carriers of derivative ileostomies present a defunctionalization of the preserved rectum and the area where the colorectal anastomosis has been performed for several months, even years. This is due to post-operative treatments with systemic chemotherapy and long waiting lists in the public health system. The patient who has already been operated on for his or her early neoplasm and needs the closure of the ileostomy and transit reconstruction is no longer a priority for the system and is often "abandoned" with his or her stoma problem.

As a result of all these months of defunctionalization, at the time of stoma closure we do not know how the patient is doing from a functional point of view in relation to defecatory function and anorectal continence. It is possible that his continence is correct but it is possible that there is some incontinence and anorectal dysfunction that are unnoticed until the moment of reconstruction. These types of alterations are well defined and are known as "anterior rectal resection syndrome" and "ultra-low resection syndrome" and both refer to this type of anorectal dysfunction that alters rectal reservoir capacity, anorectal coordination and anal continence.

Therefore, one clinical problem after this waiting time is the absence of information for the surgeon and the patient about the rectoanal function. Previously to close the ileostomy and to restore the bowel continuity it would be desirable to know whether the patient will have any problem concerning the distal function of the rectum and anus.

The existence of a transit reconstruction simulation maneuver by simulating stoma closure would be of great help in predicting which patients may have problems after ileal transit reconstruction.

Furthermore, it would be desirable to made available preoperative techniques that improves safety of ileal anastomosis, with less incidence of complications (dehiscence, stenosis), when operating on a distal ileal segment improved with the preparation by distal instillation of solutions or drugs to improve the caliber for a safer ileal anastomosis, and improvement of the villous atrophy for a better intestinal absorption.

The international PCT publication WO 2019/171335 A1 refers to a device for connecting and transferring contents between a surgically created first orifice and a surgically created second orifice is provided. The device comprises a hollow body member and first and second tubular legs, and a cap for closing the hollow body and completing a sealed pathway between the tubular legs for passively conveying contents between the first and second surgically created orifices.

Chinese utility model CN-209154851 U provides an intestinal tract double-cavity stoma bridge with a near-end intestinal canal and a far-end intestinal canal of the intestinal tract double-cavity stoma, connected together through the intestinal tract double-cavity stoma bridge.

Chinese patent application CN-107625573 A relates to an intestinal double-cavity fistulization excrement transferring device, that comprises a bent pipe made of rubber, and air bags arranged on the outer sides of two ends of the bent pipe in a surrounding mode.

### Summary of the invention

The present invention is defined in the attached independent claim, and satisfactorily solves the drawbacks of the prior art, by providing a "U-ileostomy" medical device that comprises a tube-connection between the two parts of the loop ileostomy, such that, by using this medical device it is possible to check by clinical test at a pre-surgery stage, the recto anal function with a good prediction of the functional results of the surgery before the ileostomy's closure, such that optimal candidates can be identified and the right time for surgery, as well as the improvement of preoperative techniques of the efferent or distal loop.

In particular, the invention refers to a continuity device for derivative ileostomy, that comprises three connectable tubes, namely: a collector tube suitable to be inserted inside a first bowel segment of an ileostomy (proximal afferent limb), a distal tube suitable to be inserted inside a second bowel segment of an ileostomy (distal efferent limb), and a connector tube connectable and disconectable with the collector and distal tubes. Having a device formed by three tubes that can be easily connected and disconnected, provides the advantage the device is easy to install in a patient, and any of the tubes can be easily replaced or cleaned if it gets obstructed.

The connector tube has a curved or arcuated configuration, and it is made of a deformable material or alternatively it has a compressible configuration. In a preferred embodiment, the connector tube has a U-shaped or any other curved configuration suitable for this purpose.

The above-mentioned three tubes are configured such that the connector tube can be connected and disconnected with the collector and distal tubes, so that, when the three tubes are connected during a derivative ileostomy procedure, fecal material can circulate from the first bowel segment to the second bowel segment through the collector, connection and distal tubes. For that, the tubes are dimensioned in way that the three tubes can be coupled directly, or alternatively the device incorporates connection elements for connecting and disconnecting the tubes.

The device further comprises retention means configured to retain the collector and distal tubes fixed to the bowels segments while they are in use, avoiding displacement of the device. In a preferred embodiment, these retention means comprise a first and second inflatable bags respectively mounted on the collector tube and the distal tube, such that these inflatable bags are configured to retain the respective tubes fixed to a part of the bowel segments at which are inserted when they are inflated.

In addition, the device includes first and second conduits extending respectively along the collector and distal tubes, and communicating respectively with the interior of the first and second bags. These, first and second conduits are accessible from outside the device for inflating the bags, by means of any proper pumping means like for example, a syringe.

With the "U-ileostomy" medical device for ileal reconstruction of the invention, the condition of the defunctionalized bowel segments is improved prior to surgery. Thanks to the transit of the ileal content and the irrigations trough the device it is possible to improve and optimize the diameter of the distal ileum and its absorption's capacity.

The device allows for the improvement of the ileal loop and the colon and rectum that have been defunctionalized for several months. The lack of transit leads to villous atrophy and lack of initial absorption, as well as a thinning and collapse of the digestive tract. All this condition problems when performing the ileo-ileal anastomosis due to discrepancy between the calibers of the two intestinal segments, as well as absorption problems in the first days or weeks after the reconstruction of the digestive transit.

All this would mean an improvement in the care of patients with colorectal cancer who undergo loop derivative ileostomy as a temporary derivation mechanism to alleviate an obstructive condition or to protect an anastomosis with risk of dehiscence.

By using the "U-ileostomy" device, the absence of the stoma is simulated since the ileal continuity is artificially restored. In addition, as many clinical tests or reconstruction trials as necessary in the patient can be performed, and the test can be suspended at the time simply by removing the device.

With the "U-ileostomy" transit reconstruction simulation device, the condition of the dysfunctional bowel segments can be improved prior to surgery by optimizing the caliber of the distal ileum and absorption in the colon. The device is safe for the patient and easy to use. It does not pose an additional problem to stoma management as it is inserted into the ileal segments and covered by the ileostomy bag.

### Brief description of the drawings

Preferred embodiments of the invention are henceforth described with reference to the accompanying drawings, wherein:
Figures 1 and 3.- shows schematic representations of the device of the invention, wherein arrows indicate the rectal material flow direction.
Figure 2.- shows a perspective view partially sectioned of a practical implementation of the invention.

### Preferred embodiment of the invention

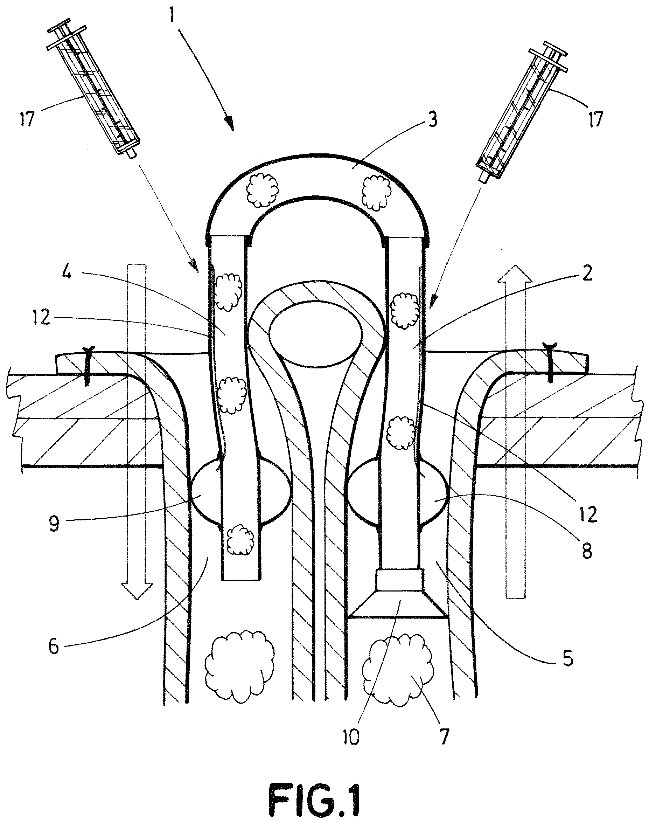

**Figure 1** shows a schematic representation of the "U-ileostomy" device (1) of the invention, that comprises a collector tube (2) suitable to be inserted inside a first bowel segment (5) of an ileostomy, a distal tube (4) suitable to be inserted inside a second bowel segment (6) of an ileostomy, and a connector tube (3) having a U-shaped configuration, and having two ends respectively coupled with a proximal end of the collector and distal tubes (2,4).

The connector tube ((3) has a U-shaped configuration in this implementation, and it is made of silicone, such that this tube can be compressed manually by a surgeon in order to force fecal material (7) to flow towards the distal tube (4).

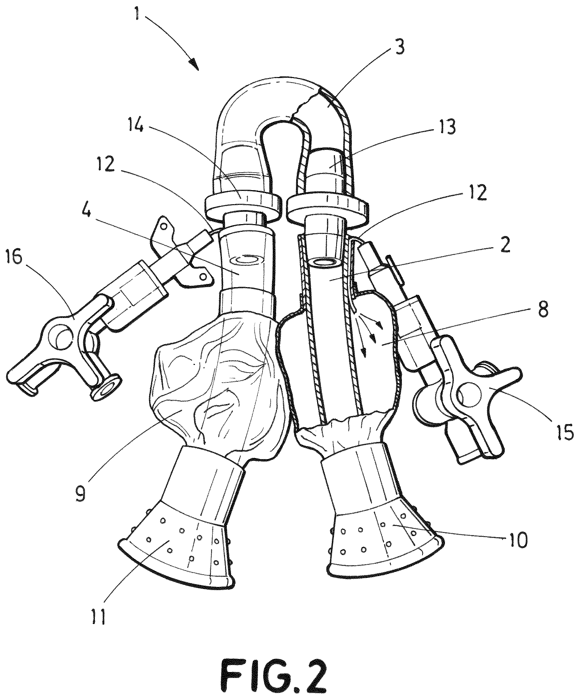

As shown in **Figure 2****,** for the connection of the three tubes, the device (1) is provided with a pair of connection elements (13), which consist of tubular bodies having one end connectable with one end of the connector tube (3) and another end connectable with a proximal end of the collector tube (2) or a proximal end of the distal tube (4). Each connection element (13) has a gripping wall (14) transversally arranged and placed between the two ends of the connection element (13), which facilities pulling of the connection element (13) to disconnect the tubes.

The connection elements (13) facilitate manual coupling and decoupling between the tubes, so as to allow an easy replacement of any of the tubes or cleaning operation, if any of the tubes get obstructed.

The device (1) further comprises first and second inflatable bags (8,9) respectively mounted on the collector tube (2) and the distal tube (4). The inflatable bags (8,9) are configured to retain the respective tubes fixed to a part of the bowel segments at which are inserted, by pressing internally on the bowels internal surface, avoiding any damage to the bowel tissue. Preferably, the inflatable bags (8,9) are made of silicone.

For inflating the bags (8,9), the device (1) incorporates first and second conduits (12) extending respectively along the collector and distal tubes (2,4) as shown in **Figure 2****.** These conduits communicate respectively with the interior of the first and second bags (8,9) with the exterior of the device, such that, first and second conduits are accessible from outside the device (1) for inflating the bags. For this purpose, the device (1) incorporates first and second conventional valves (15,16) externally placed to the device, and connected respectively with the first and second conduits (12). Each of these valves (15,16) has an opening for the connection of a syringe (17) as it is schematically represented in **Figure 1****.**

The first and second conduits (12) are joined respectively to an inner surface of the collection and distal tubes (2,4), or alternatively they are integrally formed with the collection and distal tubes.

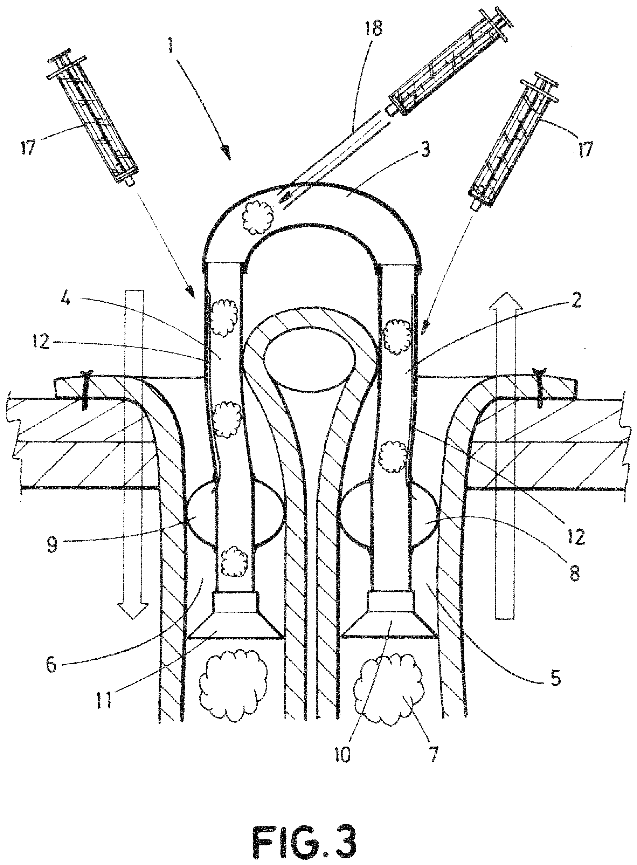

In the preferred embodiment of **Figure 3**, the device (1) includes a third conduit (18) fluidly communicated with the interior of the connector tube (3) interior, and accessible from outside the connector tube (3). This third conduit (18) can be used as irrigation tube to inject washing liquids to clean the connector and distal tubes (2,4), and to inject chemical substances into the connector tube (3) towards the second bowel segment (6). Also in this embodiment, a second funnel (11) is fitted at the distal end of the distal tube (4), in order to prevent longitudinal displacements of the distal (4).

The collection and the distal tubes (2,4) are made of a flexible material, and their distal end has a funnel configuration. Preferably and as shown in **Figure 1****,** a first frusto-conical funnel member (10) is coupled at a distal end of the collection tube (2), and optionally a second frusto-conical funnel (11) is coupled at a distal end of the distal tube (4). Both funnels (10,11) are preferably made of a silicone material, and are dimensioned to fit at the bowel segment to collect all fecal material (/ileal content) (7) into the collection tube (2), and to prevent this material from flowing between the collection and the bowel, thereby preventing that that ileal content could exert pressure on the bags (8,9) and displace the collection tube from the correct position.

As represented more clearly in **Figure 2****,** a part of each inflatable bag (8,9) is retained between the collection or distal tube (2,4) and a part of a frusto-conical funnel (10,11).

The medical device is manufactured with biocompatible and eco-friendly materials, latex-free and easy to clean and to sterilize.

Therefore, with the above described structure of the "U-ileostomy" device of the invention, at least the following advantages and functionalities are accomplished:
- the device is safe for the patient and easy to use,
- it allows checking with a clinical test the later rectoanal function with a good prediction of the functional results of the surgery before the ileostomy's closure,
- it is not an additional problem to the management of the stoma because it is introduced into the ileal segments and covered by the ileostomy bag,
- it allows selecting patients who are good candidates for closing the ileostomy because of his correct rectoanal function tested preoperatively with a clinical test with the device. With the clinical test the patient may use the defunctionalized part of the bowel and to know how the anal function will be. This selection offers the possibility to exclude for surgery the patients with an improper function. Some actions may be required to correct the defecation disorders previous to surgery in the patients with rectoanal problems detected with the test.
- it allows checking if there is any leakage or obstruction in the distal bowel before the surgery. With the installation of contrast solutions we can offer a radiologic study of the distal bowel in order to asses any problem.
- it improves the condition of the defunct bowel by preparing it for subsequent transit reconstruction surgery. In this way, a faster functional recovery of the defunct segment is achieved.
- it improves the function of the distal bowel. The distal part of the ileum and the defunctionalized colon and rectum usually have a glandular atrophy due to the absence of alimentary transit. With the irrigation of some solutions trough the device it is possible to improve the function of the ileum and colon with the pre-habilitation of the glandular tissue.

Other preferred embodiments of the present invention are described in the appended dependent claims and the multiple combinations of those claims.

## Claims

1. Continuity device (1) for derivative ileostomy suitable for preoperative predictive test of derivative ileostomy closure surgery,
the device (1) comprising:
a collector tube (2) suitable to be inserted inside a first bowel segment (5) of an ileostomy,
a distal tube (4) suitable to be inserted inside a second bowel segment (6) of an ileostomy,
wherein the device (1) further comprises a connector tube (3) having a curved configuration, and made of a deformable material,
wherein the connector tube (3) can be connected and disconnected with the collector and distal tubes (2,4), such that, when the connector tube (3) is connected, fecal material can circulate through the collector, connection and distal tubes (2,3,4),
the device (1) further comprising a first and second inflatable bags (8,9) respectively mounted on the collector tube (2) and the distal tube (4), wherein the inflatable bags (8,9) are configured to retain the respective tubes (2,4) fixed to a part of the bowel segments (5,6) at which are inserted when they are inflated;
the device further comprising first and second tubular connection elements (13) for connecting the connector tube (3) with the collector and distal tubes (2,4), wherein the tubular connection elements (13) are tubular bodies having one end connectable with one end of the connector tube (3) and another end connectable with a proximal end of the collector tube (2) or a proximal end of the distal tube (4); and
wherein the connection element (13) has a gripping wall (14) transversally arranged and placed between the two ends of the connection element.

2. Device according to claim 1, wherein the connector tube (3) has a U-shaped configuration and it is made of silicone, such that the connector tube (3) is suitable to be compressed manually by a surgeon in order to force fecal material (7) to flow towards the distal tube (4).

3. Device according to claim 1 or 2 further comprising first and second conduits (12) extending respectively along the collector and distal tubes (2,4), and communicating respectively with the interior of the first and second bags (8,9), and wherein the first and second conduits (12) are accessible from outside the device (1) for inflating the bags.

4. Device according to any of the preceding claims further comprising a first frusto-conical funnel (10) coupled at a distal end of the collection tube (2), and wherein the funnel is preferably made of silicone.

5. Device according to any of the preceding claims, further comprising a second frusto-conical funnel (11) coupled at a distal end of the distal tube (4), and wherein the funnel is preferably made of silicone.

6. Device according to any of the claims 4 or 5, wherein a part of the inflatable bag (8,9) is retained between the collection tube (2) or the distal tube (4) and a frusto-conical funnel (10,11).

7. Device according to claim 3, wherein the first and second conduits (12) are joined respectively to an inner surface of the collection and distal tubes (2,4), or they are integrally formed with the collection and distal tubes.

8. Device according to any of the preceding claims, further comprising first and second valves (15,16) externally placed to the device, and connected respectively with the first and second conduits (12), and having an opening for their connection with pumping means (17).

9. Device according to any of the preceding claims, further comprising a third conduit (18) fluidly communicated with the connector tube (3) interior and accessible from outside the connector tube (3) to inject washing liquids to clean the connector and distal tubes (2,4), and to inject chemical substances into the connector tube (3) towards the second bowel segment (6).

## Patentansprüche

1. Kontinuitätsvorrichtung (1) für ableitende Ileostomie, welche für einen präoperativen prädiktiven Test von Verschlussoperation bei ableitender Ileostomie geeignet ist,
wobei die Vorrichtung (1) Folgendes umfasst:
ein Sammelrohr (2), welches dafür geeignet ist, innerhalb eines ersten Darmsegments (5) einer Ileostomie eingeführt zu werden,
ein distales Rohr (4), welches dafür geeignet ist, innerhalb eines zweiten Darmsegments (6) einer Ileostomie eingeführt zu werden,
wobei die Vorrichtung (1) zusätzlich ein Verbindungsrohr (3) umfasst, welches eine gekrümmte Ausbildung aufweist und aus einem verformbaren Material hergestellt ist,
wobei das Verbindungsrohr (3) mit dem Sammel- und dem distalen Rohr (2, 4) verbunden werden kann und dessen Verbindung damit gelöst werden kann, sodass, wenn das Verbindungsrohr (3) verbunden ist, Fäkalmaterial durch das Sammel-, das Verbindungs- und das distale Rohr (2, 3, 4) zirkulieren kann,
wobei die Vorrichtung (1) zusätzlich einen ersten und einen zweiten aufblasbaren Beutel (8, 9) umfasst, welche jeweils am Sammelrohr (2) und am distalen Rohr (4) montiert sind, wobei die aufblasbaren Beutel (8, 9) dazu ausgebildet sind, die jeweiligen Rohre (2, 4) an einem Teil der Darmsegmente (5, 6), in welche sie eingeführt sind, fixiert zu halten, wenn sie aufgeblasen werden;
wobei die Vorrichtung zusätzlich ein erstes und ein zweites rohrförmiges Verbindungselement (13) zum Verbinden des Verbindungsrohrs (3) mit dem Sammel- und dem distalen Rohr (2, 4) umfasst, wobei die rohrförmigen Verbindungselemente (13) rohrförmige Körper sind, welche ein Ende, welches mit einem Ende des Verbindungsrohrs (3) verbindbar ist, und ein anderes Ende, welches mit einem proximalen Ende des Sammelrohrs (2) oder einem proximalen Ende des distalen Rohrs (4) verbindbar ist, aufweisen; und
wobei das Verbindungselement (13) eine Greifwand (14) aufweist, welche quer angeordnet und zwischen den beiden Enden des Verbindungselements platziert ist.

2. Vorrichtung nach Anspruch 1, wobei das Verbindungsrohr (3) eine U-förmige Ausbildung aufweist und aus Silikon hergestellt ist, sodass das Verbindungsrohr (3) dafür geeignet ist, manuell von einem Chirurgen zusammengedrückt zu werden, um Fäkalmaterial (7) dazu zu zwingen, zum distalen Rohr (4) hin zu fließen.

3. Vorrichtung nach Anspruch 1 oder 2, zusätzlich umfassend eine erste und eine zweite Leitung (12), welche sich jeweils entlang des Sammel- und des distalen Rohrs (2, 4) erstrecken und jeweils mit dem Inneren des ersten und des zweiten Beutels (8, 9) kommunizieren, und wobei die erste und die zweite Leitung (12) von außerhalb der Vorrichtung (1) zugänglich sind, um die Beutel aufzublasen.

4. Vorrichtung nach einem der vorhergehenden Ansprüche, zusätzlich umfassend einen ersten kegelstumpfförmigen Trichter (10), welcher an einem distalen Ende des Sammelrohrs (2) gekoppelt ist, und wobei der Trichter vorzugsweise aus Silikon hergestellt ist.

5. Vorrichtung nach einem der vorhergehenden Ansprüche, zusätzlich umfassend einen zweiten kegelstumpfförmigen Trichter (11), welcher an einem distalen Ende des distalen Rohrs (4) gekoppelt ist, und wobei der Trichter vorzugsweise aus Silikon hergestellt ist.

6. Vorrichtung nach einem der Ansprüche 4 oder 5, wobei ein Teil des aufblasbaren Beutels (8, 9) zwischen dem Sammelrohr (2) oder dem distalen Rohr (4) und einem kegelstumpfförmigen Trichter (10, 11) gehalten wird.

7. Vorrichtung nach Anspruch 3, wobei die erste und die zweite Leitung (12) jeweils an eine Innenfläche des Sammel- und des distalen Rohrs (2, 4) gefügt sind, oder sie mit dem Sammel- und dem distalen Rohr integral gebildet sind.

8. Vorrichtung nach einem der vorhergehenden Ansprüche, zusätzlich umfassend ein erstes und ein zweites Ventil (15, 16), welche äußerlich der Vorrichtung platziert sind und jeweils mit der ersten und der zweiten Leitung (12) verbunden sind, und welche eine Öffnung für deren Verbindung mit Pumpmitteln (17) aufweisen.

9. Vorrichtung nach einem der vorhergehenden Ansprüche, zusätzlich umfassend eine dritte Leitung (18), welche mit dem Inneren des Verbindungsrohrs (3) in Fluidkommunikation steht und von außerhalb des Verbindungsrohrs (3) zugänglich ist, um Waschflüssigkeiten einzuspritzen, um das Sammel- und das distale Rohr (2, 4) zu reinigen, und um chemische Stoffe in das Verbindungsrohr (3) zum zweiten Darmsegment (6) hin einzuspritzen.

## Revendications

1. Dispositif de continuité (1) pour iléostomie dérivée approprié pour un test prédictif préopératoire de chirurgie de fermeture d'iléostomie dérivée,
le dispositif (1) comprenant :
un tube collecteur (2) approprié pour être inséré dans un premier segment d'intestin (5) d'une iléostomie,
un tube distal (4) approprié pour être inséré dans un deuxième segment d'intestin (6) d'une iléostomie,
dans lequel le dispositif (1) comprend en outre un tube connecteur (3) présentant une configuration incurvée, et réalisé en un matériau déformable,
dans lequel le tube connecteur (3) peut être connecté et déconnecté par rapport aux tubes collecteur et distal (2, 4), de telle sorte que, lorsque le tube connecteur (3) est connecté, les matières fécales peuvent circuler à travers les tubes collecteur, connecteur et distal (2, 3, 4),
le dispositif (1) comprenant en outre un premier et un deuxième sacs gonflables (8, 9) montés respectivement sur le tube collecteur (2) et le tube distal (4), dans lequel les sacs gonflables (8, 9) sont configurés pour retenir les tubes (2, 4) respectifs fixés à une partie des segments d'intestin (5, 6) dans lesquels ils sont insérés lorsqu'ils sont gonflés ;
le dispositif comprenant en outre un premier et un deuxième éléments de connexion tubulaires (13) pour connecter le tube connecteur (3) aux tubes collecteur et distal (2, 4), dans lequel les éléments de connexion tubulaires (13) sont des corps tubulaires ayant une extrémité pouvant être connectée à une extrémité du tube connecteur (3) et une autre extrémité pouvant être connectée à une extrémité proximale du tube collecteur (2) ou à une extrémité proximale du tube distal (4) ; et
dans lequel l'élément de connexion (13) présente une paroi de préhension (14) disposée transversalement et placée entre les deux extrémités de l'élément de connexion.

2. Dispositif selon la revendication 1, dans lequel le tube connecteur (3) présente une configuration sous forme de U et il est réalisé en silicone, de telle sorte que le tube connecteur (3) est approprié pour être comprimé manuellement par un chirurgien afin de forcer les matières fécales (7) à s'écouler vers le tube distal (4).

3. Dispositif selon la revendication 1 ou 2, comprenant en outre un premier et un deuxième conduits (12) s'étendant respectivement le long des tubes collecteur et distal (2, 4), et communiquant respectivement avec l'intérieur du premier et du deuxième sacs (8, 9), et dans lequel le premier et le deuxième conduits (12) sont accessibles depuis l'extérieur du dispositif (1) pour le gonflage des sacs.

4. Dispositif selon l'une quelconque des revendications précédentes, comprenant en outre un premier entonnoir tronconique (10) couplé à une extrémité distale du tube de collecte (2), et dans lequel l'entonnoir est de préférence réalisé en silicone.

5. Dispositif selon l'une quelconque des revendications précédentes, comprenant en outre un deuxième entonnoir tronconique (11) couplé à une extrémité distale du tube distal (4), et dans lequel l'entonnoir est de préférence réalisé en silicone.

6. Dispositif selon l'une quelconque des revendications 4 ou 5, dans lequel une partie du sac gonflable (8, 9) est retenue entre le tube de collecte (2) ou le tube distal (4) et un entonnoir tronconique (10, 11).

7. Dispositif selon la revendication 3, dans lequel le premier ou le deuxième conduits (12) sont attachés respectivement à une surface interne des tubes de collecte et distal (2, 4), ou ils sont formés d'un seul tenant avec les tubes de collecte et distal.

8. Dispositif selon l'une quelconque des revendications précédentes, comprenant en outre une première et une deuxième vannes (15, 16) placées à l'extérieur du dispositif, et connectées respectivement au premier et au deuxième conduits (12), et présentant une ouverture pour leur connexion à des moyens de pompage (17).

9. Dispositif selon l'une quelconque des revendications précédentes, comprenant en outre un troisième conduit (18) en communication de fluide avec le tube connecteur (3), intérieur et accessible depuis l'extérieur du tube connecteur (3), pour injecter des liquides de lavage pour nettoyer les tubes connecteur et distal (2, 4), et pour injecter des substances chimiques dans le tube connecteur (3) vers le deuxième segment d'intestin (6).
